# EUROPEAN PATENT SPECIFICATION

(11) **EP 0 723 431 B1**
(45) Date of publication and mention of the grant of the patent: **15.07.1998**
(21) Application number: 94931244.1
(22) Date of filing: 13.10.1994
(51) Int. Cl.: A61F 13/15

(54) **SHEATHING LAMINATE FOR AN ABSORBENT PRODUCT, PROCESS FOR MANUFACTURE OF THE SHEATHING LAMINATE AND ABSORBENT PRODUCT CONTAINING SUCH SHEATHING LAMINATE**
BESCHICHTUNGSLAMINAT FÜR EIN ABSORBIERENDES PRODUKT, VERFAHREN ZUR HERSTELLUNG DES BESCHICHTUNGSLAMINATS UND ABSORBIERENDES PRODUKT MIT EINEM SOLCHEN BESCHICHTUNGSLAMINAT
STRATIFIE DE REVETEMENT POUR PRODUIT ABSORBANT, SON PROCEDE DE FABRICATION ET PRODUIT ABSORBANT CONTENANT CELUI-CI

(30) Priority: 15.10.1993 SE 9303406
(43) Date of publication of application: 31.07.1996
(73) Proprietor: SCA Mölnlycke AB, 405 03 Göteborg (SE)
(72) Inventor: LINDQUIST, Bengt, S-443 51 Lerum (SE); BÖHM, Thomas, S-421 69 Västra Frölunda (SE)
(74) Representative: Romare, Laila Anette
(86) International application number: SE9400962
(87) International publication number: WO9510254

(56) References cited:
- WO-A-93/09744
- WO-A-93/12745

## Description

The present invention relates to a sheathing laminate for an absorbent product, such as a sanitary napkin, a diaper, an incontinence protector or the like.

In absorbent products of the above-mentioned type, it is known to use as a fluid permeable outer layer, which during use is intended to face the user, a plastic net or a perforated plastic film. Such surface materials have good rewetting properties, so that the absorbent body when used is felt to have a dry and comfortable surface. A disadvantage is, however, that the edges of the net or plastic film can chafe and be felt to be sharp against the skin of the user.

To avoid this disadvantage, it has previously been suggested (see WO-A-9312745) that the edges of the net or plastic film be covered will a fold of soft, skin-friendly material, preferably a non-woven material. Making such folds presents, however, certain difficulties as does fixing the same to the net or the perforated plastic film at high production speed. Furthermore, a sheathing layer constructed in this manner cannot be prepared ahead of time and stored intermediately in a roll.

In order to eliminate the above-mentioned difficulties of the known technology in the field, it is suggested according to the present invention that the laminate comprises a web of plastic film to a first side of which, being that side which is intended to face the user's body, there are fixed two spaced, longitudinal strips of non-woven material, while to the other second side of the plastic web - in an area between the two strips on said first side of the plastic film web - there is fixed a longitudinal intermediate strip of non-woven material. This provides a surface material which has soft textile lateral edge portions which do not need to be folded over edge portions of the net or the perforated plastic film and which during manufacture can be rolled up into a roll for intermediate storage prior to manufacture of the absorbent products themselves.

In order to facilitate rolling up into an essentially homogeneous roller, the intermediate strip has a width corresponding to the space between the spaced strips at the same time as all of the strips have preferably the same thickness.

In a preferred embodiment of the sheathing laminate according to the invention, the spaced strips extend along the lateral edges of the plastic film web and can be joined by glueing, welding, heat calendering or the like to the lateral edges of a fluid impermeable sheathing layer forming the outer side, i.e. the side of the absorbent product facing away from the user.

According to an additional embodiment of the present invention, at least one of the separate spaced strips is located spaced inside the adjacent lateral edge of the plastic film web, thus forming the free lateral edge zone of the plastic film web which is unperforated. Thus, the sheathing laminate can completely envelope an absorbent body and form the fluid impermeable sheathing layer which faces away from the user. Alternatively, the width of said strips spaced from each other can be substantially greater than the width of the intermediate strip, and the sheathing laminate can in this case as well completely envelope the absorbent body, but in this case provide a soft textile surface on the outside of the final product as well.

The intermediate strip, which is intended to be disposed on the side of the plastic film web which faces away from the user, has good fluid conducting properties in order to divert liquid to an adjacent absorbent body of the final product.

In order to make the sheathing laminate fluid permeable when used as a top layer on an absorbent product, the laminate has perforations at least within a portion of the area of the intermediate strip. These perforations can have varied shape and/or size in a manner suitable for the purpose.

The invention also relates to a process for manufacture of a sheathing laminate for absorbent products, such as sanitary napkins, diapers, incontinence protectors and the like, which process is characterized in that at least one pair but preferably a plurality of pairs of mutually spaced, longitudinal strips of non-woven material are moved towards and fixed to one side of a plastic film web, that an additional longitudinal intermediate strip of non-woven material is fixed to the other side of the plastic film web on portions thereof which lie between respective pairs of the mutually spaced strips on the opposite side.

It is suitable that at least one of the strips of non-woven material be moved towards and fixed to the plastic film web in semi-molten state. Alternatively, the strip can be fixed to various places along the plastic film web and with other adhesive methods, such as glueing, heat calendering, ultrasonic welding or the like.

In order to make the sheathing laminate liquid permeable when used as a top layer on an absorbent product, the sheathing laminate is perforated at least within a portion of the area for each intermediate strip.

Finally, the invention emcompasses an absorbent product, such as a sanitary napkin, a diaper, an incontinence protector or the like, which comprises an absorbent body enveloped between a liquid impermeable bottom layer and a liquid permeable top layer, which when the product is used faces the body of the user, whereby according to the invention the top layer is a laminate, comprising a longitudinal plastic film piece, to a first side of which, being that side which is intended to face the user's body, there are fixed two mutually spaced longitudinal strips of non-woven material, extending over at least the lateral edge portion of the product, while to the other second side of the plastic film piece there is fixed an additional longitudinal strip of non-woven material in the liquid absorbing area between the two steps on said first opposite side of the plastic film piece, said laminate having perforations at least within a portion of the area for the intermediate non-woven strip.

Additional features and advantages of the present invention will be revealed in more detail below with reference to the accompanying drawings, of which:
Fig. 1 shows schematically the principle for manufacturing a sheathing laminate according to the invention;
Fig. 2 shows a cross-section through the laminate web made in Fig. 1;
Fig. 3 shows a plan view of a sheathing laminate piece with indicated placement of the absorbent body;
Fig. 4 shows an embodiment of an absorbent product with a sheath laminate according to the invention and with attachment tabs integrated with the laminate;
Fig. 5 shows a cross-section taken along the line V-V in Fig. 4; and
Fig. 6 shows a cross-section through an alternative embodiment of an absorbent product according to the invention.

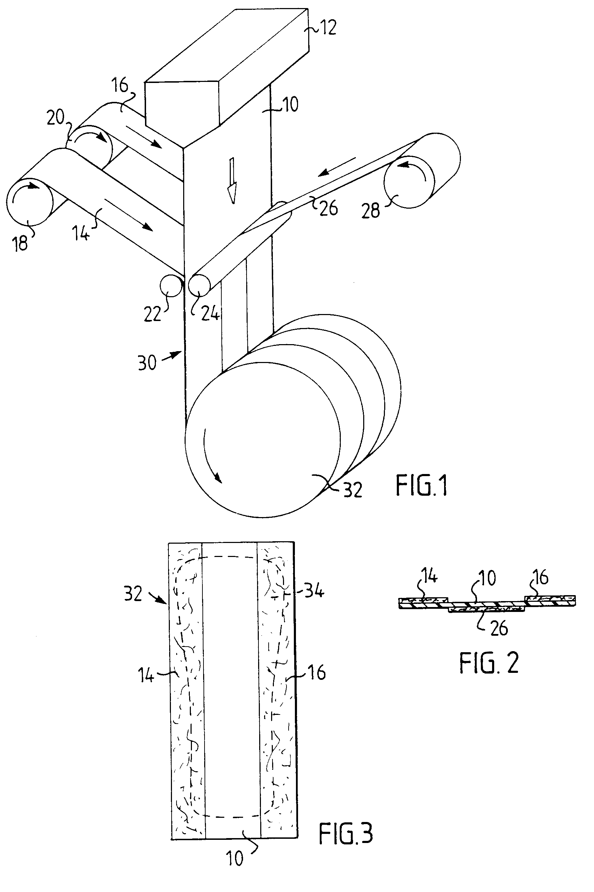

Fig. 1 shows schematically the principle of manufacturing a laminate web from which there are to be made individual fluid permeable sheathing layers for fluid absorbent products, such as sanitary napkins, diapers, incontinence protectors and the like.

A plastic film web 10 is formed by an extrusion die 12 and moves vertically downwards. The plastic film web 10 is laminated on its one side with two mutually spaced strips 14 and 16 of non-woven material suitable for the purpose. In the example shown, these strips are unwound from respective storage rolls 18 and 20 and are fixed to the edge portions of the plastic film web 10 which is in a semi-molten state, with the aid of press rollers 22 and 24, while the other side of the plastic film web 10 is laminated at the same time with a third strip 26 of non-woven material, which is wound off from a roll 28, the width of said third strip essentially corresponding to the distance between the strips 14 and 16. The sheathing laminate web 30 thus formed is wound unto a roll 32 and can be intermediately stored thereon, before the web 30 in the subsequent manufacture of individual absorbent products, is cut into separate sheathing layers, which are to form the top layer, i.e. the side facing the user, of the final product.

The mutually spaced strips 14, 16 of non-woven material are intended to form comfortably soft, skin-contacting textile edge portions of the final product.

The non-woven strip 26 should, however, have the property of being able to conduct fluid from the wearer of the absorbent product to an absorbent body lying inside, and be able to visually mask the absorbed fluid. At the same time, the intermediate strip 26 serves as a spacing layer between the user and the fluid which has been absorbed into the absorbent body lying inside the product.

Suitable materials for use in the non-woven strips 14, 16 and 26 are, for example, fibres of synthetic polymers, such as polypropylene, polyester and polyethylene, individually or in the form of mixtures, co-polymers or hi-component fibres. Non-woven materials including cellulose based fibres such as rayon, cotton, cellulose fluff or the like, can also be used. The non-woven material can also be made by any known method which is suitable for the purpose.

It is suitable that the strips 14, 16 and 26 have essentially the same thickness, which facilitates winding up of the laminate web 30 into a roll 32 for intermediate storage.

In order to make the sheathing laminate permeable to fluid when used as a top layer in an absorbent product, the laminate is provided with a perforation at least within the area of the central portion of the absorbent product, preferably within the area coinciding with the intermediate non-woven strip 26 lying along the central portion. The perforations can have varying shape and size within various areas of the sheathing laminate. The perforation can also be of the type described in SE-C-8402614-5, and can be made either after the lamination but prior to rolling up of the roll 32, or in connection with the manufacture of the final product, i.e. the absorbent product.

Despite the fact that Fig. 1 shows the manufacture of a single sheathing laminate web 30, it is possible within the scope of the invention and suitable to simultaneously manufacture a number of sheathing laminate webs 30 in parallel, i.e. on a common width of plastic film web to laminate a plurality of pairwise mutually spaced non-woven strips 14, 16, on one side of the web and intermediate strips 26 at intended locations on the other side. Such a broad multiple web can then be cut longitudinally into a plurality of separate laminate webs 30, either prior to winding up into rolls 32 or at the subsequent manufacture of the final product, which can be done in direct connection with the manufacture of the laminate in a so-called in-line-production.

Fig. 3 shows a plan view of a piece of sheathing laminate 32 cut out of the laminate web 30, where the placement of the underlying absorbent body 34 is indicated.

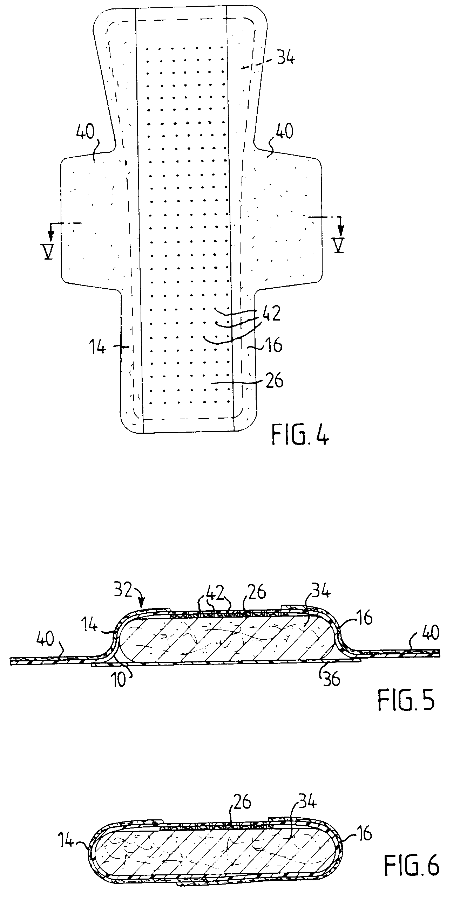

Figs. 4 and 5 show in more detail an embodiment of an absorbent body (sanitary napkin) with a fluid permeable sheathing laminate according to the invention as a top layer, i.e. the sheathing layer of the product lying closest to the body. The sanitary napkin comprises a conventional longitudinal absorbent body 34 preferably of fluff pulp with or without mixed-in so-called super-absorbents, which are enclosed between a liquid impermeable bottom layer 36 of suitable plastic material and a fluid permeable top layer of the sheathing laminate 32 according to the present invention. Other known materials for the absorbent body and the bottom layer can of course be used within the scope of the invention. The top layer is thus made of a plastic film 10 with a central strip 26 of non-woven material placed between the plastic film 10 and the absorbent body 34, and two lateral strips 14, 16 of non-woven material applied on the outside of the plastic film 10 at the edge portions of the napkin. The plastic film 10 is joined in a suitable manner to the bottom layer 36, for example by glueing or ultrasonic welding. The lateral strips 14, 16 can be made with attachment tabs or wings 40 extending laterally to facilitate fixing of the sanitary napkin in the crotch of a pair of underpants. The wings 40 can thus be formed in one piece with the strip 14, 16, or separate wings can be joined in a suitable manner to the strips 14, 16 or with the plastic film 10 or the bottom layer 36.

At least in the fluid absorbing zone of the sanitary napkin, i.e. the area of the intermediate strip 26, perforations 42 of suitable shape and distribution are made through the plastic film web 10 and the strip 26 to conduct bodily fluid into the absorbent body 34 of the sanitary napkin.

Fig. 6 shows an additional embodiment of an absorbent body with a sheathing laminate according to the present invention. In this embodiment, the plastic film 10 and the lateral strips 14, 16 have been made so wide that the laminate sheathing itself can envelop the entire absorbent body 34. Alternatively, the lateral strips 14, 16 of non-woven material can be made less broad, so that only the plastic film 10 can form the fluid impermeable bottom layer of the product.

## Claims

1. Sheathing laminate for an absorbent product, such as a sanitary napkin, a diaper, an incontinence protector or the like, **characterized** in that the laminate comprises a web (10) of plastic film to a first side of which, being that side which is intended to face the user's body, there are fixed two mutually spaced, longitudinal strips (14, 16) of non-woven material, while to the other second side of the plastic film web (10) - in an area between the two strips on said first side of the plastic film web - there is fixed a longitudinal intermediate strip (26) of non-woven material.

2. Sheathing laminate according to Claim 1, **characterized** in that the intermediate strip (26) has a width corresponding to the distance between the spaced strips (14, 16).

3. Sheathing laminate according to Claim 1 or 2, **characterized** in that the spaced strips (14, 16) extend along the lateral sides of the plastic film web (10).

4. Sheathing laminate according to Claim 1 or 2, **characterized** in that at least one of the mutually spaced strips (14, 16) is spaced inside the adjacent lateral edge of the plastic film web (10).

5. Sheathing laminate according to one of Claims 1-4, **characterized** in that the mutually spaced strips (14, 16) and the intermediate strip (26) of non-woven material have essentially the same thickness.

6. Sheathing laminate according to one of Claims 1-5, **characterized** in that the width of said mutually spaced strips (14, 16) is substantially greater than the width of the intermediate strip (26).

7. Sheathing laminate according to one of Claims 1-6, **characterized** in that the mutually spaced strips (14, 16) of non-woven material have super-absorbent, gel-forming properties.

8. Sheathing laminate according to one of Claims 1-7, **characterized** in that the intermediate non-woven strip (26) disposed on the inside of the plastic film piece has good fluid conducting properties.

9. Sheathing laminate according to one of Claims 1-8, **characterized** in that it has perforations (42) at least within a portion of the area for the intermediate strip (26).

10. Process for manufacture of a sheathing laminate for absorbent products, such as sanitary napkins, diapers, incontinence protectors or the like, **characterized** in that at least one pair, but preferably a plurality of pairs, of mutually spaced, longitudinal strips (14, 16) of non-woven material are moved towards and fixed to one side of a plastic film web (10), that an additional longitudinal intermediate strip (26) of non-woven material is fixed to the other side of the plastic film web (10) on portions thereof which lie between respective pairs of the mutually spaced strips (14, 16) on the opposite side.

11. Process according to Claim 10, **characterized** in that at least one of the strips (14, 16, 26) of non-woven material is moved towards and fixed to the plastic film web in a semi-molten state of the plastic film web.

12. Process according to Claim 10 or 11, **characterized** in that the laminate web (30) formed is intermediately stored by rolling up prior to manufacture of the absorbent products.

13. Process according to one of Claims 10-12, **characterized** in that the sheathing laminate is perforated at least within a portion of the area for each intermediate strip (26).

14. Absorbent product, such as a sanitary napkin, a diaper, an incontinence protector or the like, comprising an absorbent body enclosed between a fluid impermeable bottom layer (36) and a fluid permeable top layer (32), which during use of the product faces the user's body, **characterized** in that the top layer (32) consists of a laminate (30), comprising a longitudinal plastic film piece (10), to a first side of which, being that side which is intended to face the user's body, there are fixed two mutually spaced longitudinal strips (14, 16) of non-woven material extending over at least the lateral edge portion of the product, while to the other second side of the plastic film piece (10) there is fixed an additional longitudinal strip (26) of non-woven material in the liquid absorbing area between the two strips (14, 16) on said first opposite side of the plastic film piece (10), said laminate having perforations (42) at least within a portion of the area for the intermediate non-woven strip (26).

15. Product according to Claim 14, **characterized** in that the intermediate non-woven strip (26) disposed on the inside of the plastic film piece (10) has good fluid conducting properties.

16. Product according to Claim 14 or 15, **characterized** in that the two longitudinal, opposite lateral edges of the product each have a laterally projecting fixing tab (40) integrated with the sheathing laminate.

## Patentansprüche

1. Umkleidungslaminat für ein absorbierendes Produkt, wie z.B. eine Hygienebinde, eine Windel, einen Inkontinenzschutz oder ähnliches,
dadurch **gekennzeichnet,** daß
das Laminat eine Bahn (10) aus Plastikfolie aufweist, an deren erste Seite, wobei es sich um diejenige Seite handelt, die dafür vorgesehen ist, zu dem Körper des Benutzers gerichtet zu sein, zwei voneinander beabstandete Längsstreifen (14, 16) aus Vliesmaterial befestigt sind, während an die andere, zweite Seite der Plastikfolienbahn (10) - in einem Bereich zwischen den beiden Streifen an der ersten Seite der Plastikfolienbahn - ein Längszwischenstreifen (26) aus Vliesmaterial befestigt ist.

2. Umkleidungslaminat nach Anspruch 1,
dadurch **gekennzeichnet,** daß
der Zwischenstreifen (26) eine Breite aufweist, die dem Abstand zwischen den beabstandeten Streifen (14, 16) entspricht.

3. Umkleidungslaminat nach Anspruch 1 oder 2,
dadurch **gekennzeichnet,** daß
die beabstandeten Streifen (14, 16) sich entlang der seitlichen Seiten der Plastikfolienbahnen (10) erstrecken.

4. Umkleidungslaminat nach Anspruch 1 oder 2,
dadurch **gekennzeichnet,** daß
wenigstens einer der voneinander beabstandeten Streifen (14, 16) nach innen von dem benachbarten Seitenrand der Plastikfolienbahn (10) beabstandet ist.

5. Umkleidungslaminat nach einem der Ansprüche 1 bis 4,
dadurch **gekennzeichnet,** daß
die voneinander beabstandeten Streifen (14, 16) und der Zwischenstreifen (26) aus Vliesstoff im wesentlichen die gleiche Dicke aufweisen.

6. Umkleidungslaminat nach einem der Ansprüche 1 bis 5,
dadurch **gekennzeichnet,** daß
die Breite der voneinander beabstandeten Streifen (14, 16) im wesentlichen größer als die Breite des Zwischenstreifens (26) ist.

7. Umkleidungslaminat nach einem der Ansprüche 1 bis 6,
dadurch **gekennzeichnet,** daß
die beiden voneinander beabstandeten Streifen (14, 16) aus Vliesmaterial superabsorbierende, Gel ausbildende Eigenschaften aufweisen.

8. Umkleidungslaminat nach einem der Ansprüche 1 bis 7,
dadurch **gekennzeichnet,** daß
der Vlies-Zwischenstreifen (26), der an der Innenseite des Stücks der Plastikfolie angeordnet ist, gute Flüssigkeitsleitungseigenschaften aufweist.

9. Umkleidungslaminat nach einem der Ansprüche 1 bis 8,
dadurch **gekennzeichnet,** daß
dieses Perforationen (42) wenigstens innerhalb eines Abschnitts des Bereichs für den Zwischenstreifen (26) aufweist.

10. Verfahren zur Herstellung eines Umkleidungslaminats für Absorptionsprodukte, wie z.B. Hygienebinden, Windeln, Inkontinenzschutzeinrichtungen und ähnliches,
dadurch **gekennzeichnet,** daß
wenigstens ein Paar, bevorzugt mehrere Paare von voneinander beabstandeten Längsstreifen (14, 16) aus Vliesmaterial zu einer Seite einer Plastikfolienbahn bewegt und daran befestigt werden, daß ein zusätzlicher Längs-Zwischenstreifen (26) aus Vliesmaterial an die andere Seite der Plastikfolienbahn (10) an Abschnitten derselben befestigt wird, die zwischen jeweiligen Paaren der voneinander beabstandeten Streifen (14, 16) an der entgegengesetzten Seite liegen.

11. Verfahren nach Anspruch 10,
dadurch **gekennzeichnet,** daß
wenigstens einer der Streifen (14, 16, 26) aus Vliesmaterial zu der Plastikfolienbahn in einem halbgeschmolzenen Zustand der Plastikfolienbahn bewegt und daran befestigt wird.

12. Verfahren nach Anspruch 10 oder 11,
dadurch **gekennzeichnet,** daß
die ausgebildete Laminatbahn (30) zwischenzeitlich dadurch gelagert wird, daß sie vor der Herstellung der absorbierenden Produkte aufgerollt wird.

13. Verfahren nach einem der Ansprüche 10 bis 12,
dadurch **gekennzeichnet,** daß
das Umkleidungslaminat wenigstens innerhalb eines Abschnitts des Bereichs für einen jeden der Zwischenstreifen (26) perforiert ist.

14. Absorbierendes Produkt, wie z.B. Hygienebinde, Windel, Inkontinenzschutz oder ähnliches mit einem Absorptionskörper, der zwischen einer flüssigkeitsundurchlässigen Unterlage (36) und einer flüssigkeitsdurchlässigen Oberlage (32) eingeschlossen ist, die während der Verwendung des Artikels zu dem Körper des Benutzers gerichtet ist,
dadurch **gekennzeichnet,** daß
die Oberlage (32) aus einem Laminat (30) besteht, das ein Längs-Stück (10) aus Plastikfolie aufweist, an deren erste Seite, wobei es sich um diejenige Seite handelt, die dafür vorgesehen ist, zu dem Körper des Benutzers gerichtet zu sein, zwei voneinander beabstandete Längsstreifen (14, 16) aus Vliesmaterial befestigt sind, die sich über wenigstens den seitlichen Randabschnitt des Artikels erstrecken, während an die andere, zweite Seite des Stücks (10) aus Plastikfolie ein zusätzlicher Längsstreifen (26) aus Vliesmaterial in dem flüssigkeitsabsorbierenden Bereich zwischen den beiden Streifen (14, 16) an der ersten, gegenüberliegenden Seite des Stücks (10) aus Plastikfolie befestigt ist, wobei das Laminat Perforationen (42) wenigstens innerhalb eines Abschnitts des Bereichs für den Zwischenstreifen (26) aus Vliesmaterial aufweist.

15. Produkt nach Anspruch 14,
dadurch **gekennzeichnet,** daß
der Zwischenstreifen (26) aus Vliesmaterial, der an der Innenseite des Stücks (10) der Plastikfolie angeordnet ist, gute Flüssigkeitsleitungseigenschaften aufweist.

16. Produkt nach Anspruch 14 oder 15,
dadurch **gekennzeichnet,** daß
die beiden längsverlaufenden, entgegengesetzten Seitenränder des Artikels jeweils eine seitlich vorstehende Befestigungslasche (40) aufweisen, die in das Umkleidungslaminat integriert ist.

## Revendications

1. Stratifié de revêtement pour un produit absorbant comme une serviette hygiénique, une couche-culotte, une protection anti-incontinence ou analogue, caractérisé en ce que le stratifié comprend une pièce (10) de film plastique sur un premier côté de laquelle, le côté destiné à se trouver face au corps de l'utilisateur, sont fixées deux bandes longitudinales (14, 16) de matériau non tissé, espacées l'une de l'autre, tandis que sur le deuxième côté de la pièce de film plastique (10), dans une région comprise entre les deux bandes situées sur ledit premier côté de la pièce de film plastique, est fixée une bande longitudinale intermédiaire (26) en matériau non tissé.

2. Stratifié de revêtement selon la revendication 1, caractérisé en ce que la bande intermédiaire (26) a une largeur qui correspond à la distance entre les bandes espacées (14, 16).

3. Stratifié de revêtement selon la revendication 1 ou 2, caractérisé en ce que les bandes espacées (14, 16) s'étendent le long des côtés latéraux de la pièce de film plastique (10).

4. Stratifié de revêtement selon la revendication 1 ou 2, caractérisé en ce que l'une au moins des bandes espacées (14, 16) est située à une certaine distance vers l'intérieur du bord latéral adjacent de la pièce de film plastique (10).

5. Stratifié de revêtement selon l'une des revendications 1 a 4, caractérisé en ce que les bandes espacées (14, 16) et la bande intermédiaire (26) en matériau non tissé ont sensiblement la même épaisseur.

6. Stratifié de revêtement selon l'une des revendications 1 à 5, caractérisé en ce que la largeur desdites bandes espacées (14, 16) est sensiblement supérieure à la largeur de la bande intermédiaire (26).

7. Stratifié de revêtement selon l'une des revendications 1 à 6, caractérisé en ce que les bandes espacées (14, 16) en matériau non tissé ont des propriétés superabsorbantes, de formation de gel.

8. Stratifié de revêtement selon l'une des revendications 1 à 7, caractérisé en ce que la bande intermédiaire non tissée (26), placée à l'intérieur de la pièce de film plastique, a de bonnes propriétés de conduction des fluides.

9. Stratifié de revêtement selon l'une des revendications 1 à 8, caractérisé en ce qu'il comporte des perforations (42), au moins dans une partie de la région destinée à la bande intermédiaire (26).

10. Procédé de fabrication d'un stratifié de revêtement pour produits absorbants comme des serviettes hygiéniques, des couches-culottes, des protections anti-incontinence ou analogues, caractérisé en ce qu'au moins une paire, mais de préférence plusieurs paires, de bandes longitudinales (14, 16) en matériau non tissé, espacées les unes des autres, sont déplacées vers une face d'une pièce de film plastique (10) et fixées à cette dernière, en ce qu'une bande longitudinale intermédiaire supplémentaire (26), en matériau non tissé, est fixée à l'autre face de la pièce de film plastique (10), sur les parties de celle-ci qui sont situées entre les paires respectives de bandes espacées (14, 16) se trouvant sur l'autre face.

11. Procédé selon la revendication 10, caractérisé en ce que l'une au moins des bandes (14, 16, 26) de matériau non tissé est déplacée vers la pièce de film plastique et fixée à cette dernière alors que la pièce de film plastique est dans un état de semi-fusion.

12. Procédé selon la revendication 10 ou 11, caractérisé en ce que la bande stratifiée (30) ainsi formée est immédiatement stockée en bobines avant la fabrication des produits absorbants.

13. Procédé selon l'une des revendications 10 à 12, caractérisé en ce que le stratifié de revêtement est perforé, au moins dans une partie de la région destinée à chaque bande intermédiaire (26).

14. Produit absorbant comme une serviette hygiénique, une couche-culotte, une protection anti-incontinence ou analogue, comprenant un corps absorbant enfermé entre une couche inférieure (36) imperméable aux fluides et une couche supérieure (32) perméable aux fluides qui, lors de l'utilisation du produit, fait face au corps de l'utilisateur, caractérisé en ce que la couche supérieure (32) est faite d'un stratifié (30) comprenant une pièce longitudinale de film plastique (10) à un premier côté de laquelle, le côté destiné à se trouver face au corps de l'utilisateur, sont fixées deux bandes longitudinales (14, 16) de matériau non tissé, espacées l'une de l'autre, qui s'étendent au moins sur la partie formant bord latéral du produit, tandis que sur la deuxième face de la pièce de film plastique (10) est fixée une bande longitudinale supplémentaire (26), en matériau non tissé, dans la région d'absorption du liquide comprise entre les deux bandes (14, 16) situées sur ledit premier côté opposé de la pièce de film plastique (10), ledit stratifié comportant des perforations (42), au moins dans une partie de la région destinée à la bande intermédiaire non tissée (26).

15. Produit selon la revendication 14, caractérisé en ce que la bande intermédiaire non tissée (26), placée sur l'intérieur de la pièce de film plastique (10), a de bonnes propriétés de conduction des fluides.

16. Produit selon la revendication 14 ou 15, caractérisé en ce que les deux bords longitudinaux latéraux opposés du produit comportent chacun une patte de fixation (40) qui dépasse latéralement, intégrée au stratifié de revêtement.
